# EUROPÄISCHE PATENTSCHRIFT

(11) **EP 1 472 983 B1**
(45) Veröffentlichungstag und Bekanntmachung des Hinweises auf die Patenterteilung: **24.01.2007**
(21) Anmeldenummer: 04004272.3
(22) Anmeldetag: 25.02.2004
(51) Int. Cl.: A61B 17/86

(54) **Knochenverankerungselement mit Gewinde zum Herausschrauben**
Threaded bone anchoring element for unscrewing
Element d'ancrage osseux fileté comprenant un dévissage

(30) Priorität: 30.04.2003 DE 10319781
(43) Veröffentlichungstag der Anmeldung: 03.11.2004
(73) Patentinhaber: BIEDERMANN MOTECH GmbH, 78054 VS-Schwenningen (DE)
(72) Erfinder: Biedermann, Lutz, 78048 VS-Villingen (DE); Rapp, Helmar, 78652 Deisslingen (DE)
(74) Vertreter: Hofer, Dorothea

(56) Entgegenhaltungen:
- EP-A- 0 714 643
- US-A- 5 098 435
- US-A1- 2002 123 752

## Beschreibung

Die vorliegende Erfindung bezieht sich auf ein Knochenverankerungselement zum Verankern einer mechanischen Vorrichtung in einem Knochen.

Ein solches Knochenverankerungselement ist in der DE 42 34 118 in Form einer Pedikelschraube beschrieben. Es umfasst einen Schaft und einen mit einer Stange verbindbaren Kopf. Der Schaft ist mit einem Gewinde zum Einschrauben in einen Knochen versehen.

Die DE 43 07 576 beschreibt ein Knochenverankerungselement mit einer polyaxialen Verbindung zwischen dem Kopf einer Knochenschraube und einer Stange.

Bei den Knochenverankerungselementen der beschriebenen Art erfolgt das Einbringen in den Knochen durch Einschrauben, was ein relativ zeitaufwendiger und Kraft erfordernder Vorgang ist. Zudem können beim klassischen Einschrauben hohe Druckkräfte auf den Knochen ausgeübt werden, was bei älteren oder vorgeschädigten Knochen nicht wünschenswert ist.

Deshalb ist für bestimmte klinische Anforderungen, insbesondere in der Kinderchirurgie, im Halswirbelbereich oder in der Neurochirurgie, das klassische Einschrauben weniger geeignet.

Der vorliegenden Erfindung liegt daher die Aufgabe zugrunde, ein Knochenverankerungselement bereitzustellen, insbesondere für den Einsatz in der Kinderchirurgie, im Halswirbelbereich oder in der Neurochirurgie, das schneller, einfacher und mit weniger Kraftaufwand in den Knochen einzubringen ist, keine schädigenden Kräfte auf den Knochen ausübt und dennoch einen sicheren Halt gewährleistet.

Diese Aufgabe wird gelöst durch ein Knochenverankerungselement gemäß Anspruch 1. Weiterbildungen der Erfindung sind in den Unteransprüchen gekennzeichnet.

Durch das Ausbilden einer knochengewindefreien Fläche ist es möglich, das Knochenverankerungselement ohne zu schrauben zuerst in ein vorher in den Knochen eingebrachtes Befestigungsloch einzudrücken oder einzubringen und dann durch Drehen um einen vorbestimmten Winkel zuverankern.

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine perspektivische Ansicht eines Knochenverankerungselements nach einer ersten Ausführungsform der vorliegenden Erfindung;
- Fig. 2: eine Draufsicht auf die Spitze des Knochenverankerungselements nach der ersten Ausführungsform;
- Fig. 3: eine Seitenansicht auf das Knochenverankerungselement nach der ersten Ausführungsform aus der in Fig. 2 gezeigten Richtung A;
- Fig. 4: eine Seitenansicht auf das Knochenverankerungselement nach der ersten Ausführungsform aus der in Fig. 2 gezeigten Richtung B;
- Fig. 5: eine schematische Darstellung der Funktion des Knochenverankerungselements nach der ersten Ausführungsform;
- Fig. 6: eine Draufsicht auf die Spitze eines Knochenverankerungselements nach einer zweiten Ausführungsform;
- Fig. 7: eine perspektivische Ansicht eines Knochenverankerungselements nach einer dritten Ausführungsform;
- Fig. 8: eine perspektivische Ansicht eines Knochenverankerungselements nach einer vierten Ausführungsform;
- Fig. 9: eine Draufsicht auf die Spitze des Knochenverankerungselements nach der vierten Ausführungsform;
- Fig. 10: eine schematische Darstellung der Funktion des Knochenverankerungselements nach der vierten Ausführungsform;
- Fig. 11: eine schematische Darstellung einer monoaxialen Verbindung eines Knochenverankerungselements mit einem Stab;
- Fig. 12: eine schematische Darstellung einer polyaxialen Verbindung eines Knochenverankerungselements mit einem Stab;

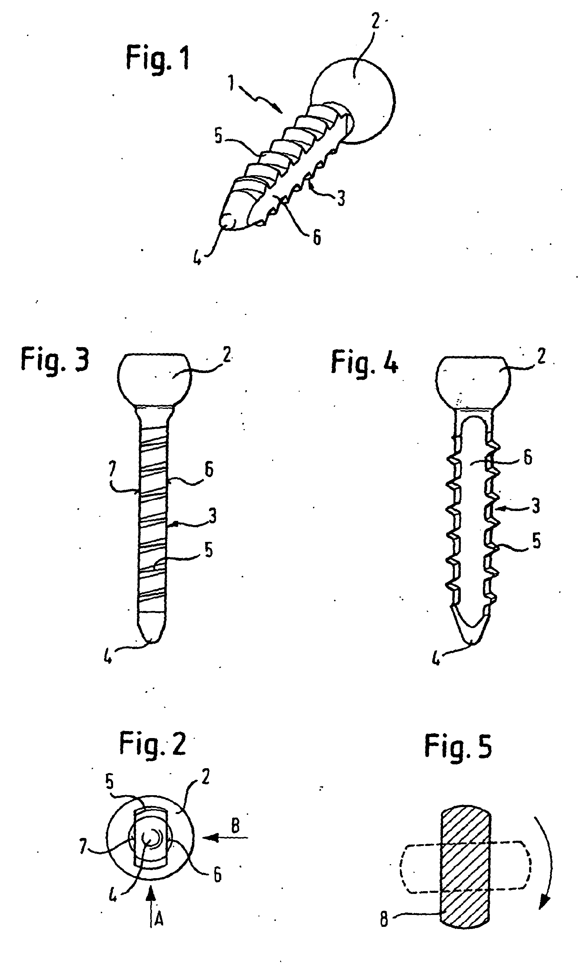

Im Folgenden wird mit Bezug auf Fig. 1 bis 5 ein Knochenverankerungselement nach einer ersten Ausführungsform der vorliegenden Erfindung beschrieben.

Wie am besten aus Fig. 1 ersichtlich weist das Knochenverankerungselement 1 einen Kopf 2 und ein Schaft 3 auf. Der Kopf 2 ist kugelsegmentförmig ausgebildet und weist eine (nicht dargestellte) Vorrichtung zum An- oder Eingreifen eines Drehwerkzeugs auf, z.B. einen Schlitz, einen Kreuzschlitz, einen Innenoder Außensechskant oder Ähnliches.

Der Schaft 3 weist an seinem dem Kopf gegenüberliegenden Ende eine Spitze 4 auf. Über die gesamte Länge des Schafts erstreckt sich ein Knochengewinde 5.

Auf zwei einander gegenüberliegenden Seiten des Schafts 3 sind parallel zueinander und zu der Schaftachse (Längsachse des Schafts 3) zwei ebene Flächen 6, 7 über die gesamte Länge des Schafts ausgebildet. Der Abstand der Flächen 6, 7 voneinander ist kleiner als der Kerndurchmesser des Knochengewindes 5, so dass die Flächen 6, 7 das Knochengewinde 5 unterbrechen.

Wie am besten aus Fig. 2 ersichtlich erhält der Schaft 3 dadurch einen annähernd rechteckförmigen Querschnitt mit durch das Gewinde gebildeten konkaven Schmalseiten. Eine erste Abmessung des Querschnitts ist durch den Abstand der Flächen 6, 7 bestimmt und eine zweite Abmessung in einer dazu senkrechten Richtung durch den Außendurchmesser des Knochengewindes 5.

Das Verankerungselement 1 ist aus Stahl, Titan oder einem anderen körperverträglichen und hinreichend stabilen Material ausgebildet.

Zum Einsetzen des Verankerungselements 1 wird zunächst, wie am besten aus Fig. 5 ersichtlich, in den Knochen ein entsprechendes Langloch 8 eingebracht. Das kann zum Beispiel durch Bohren eines Doppellochs und anschließendes Entfernen der Trennwand mit einer Raspel geschehen. Alternativ dazu kann das Langloch auch mit Hilfe einer Querfräse oder Profilfräse erzeugt werden. Das Profil des Langlochs 8 entspricht annähernd dem Querschnitt des Schafts 3. Es kann jedoch auch etwas kleiner sein.

In das Langloch 8 wird anschließend der Schaft 3 des Verankerungselements 1 durch Eindrücken oder Einklopfen so eingesetzt, dass die Längsachse des Querschnitts durch den Schaft 3 annähernd mit der Längsachse des Langlochs 8 übereinstimmt. In diesem Zustand sitzt das Verankerungselement 1 nur so fest in dem Langloch 8, daß es durch Ziehen wieder entfernt werden kann.

Anschließend wird das Verankerungselement 1 mit einem Drehwerkzeug, das an oder in dem Kopf an- bzw. eingreift, um einen vorbestimmten Winkel gedreht, vorzugsweise um 90°, was in Fig. 5 durch die gestrichelte Linie dargestellt ist. Dadurch greifen die Zähne des Knochengewindes 5 in den Knochen ein und geben dem Verankerungselement 1 einen festen Sitz.

Durch Weiterdrehen bis zu einem Winkel von 180° oder Zurückdrehen in die Ausgangsstellung kann das Verankerungselement 1 bei Bedarf wieder gelöst und aus dem Knochen herausgezogen werden.

Im Lauf der Zeit wächst der Knochen in die Freiräume ein und gibt dem Verankerungselement 1 damit einen zusätzlichen Halt.

Nach längerer Zeit, wenn das Verankerungselement 1 bereits fest von Knochen umwachsen ist, kann in bestimmten Fällen eine Entfernung des Verankerungselements 1 aus dem Knochen erforderlich sein, z.B. wenn die zu verankernde Vorrichtung entfernt werden soll. Ein einfaches Zurückdrehen und Herausziehen ist dann nicht mehr möglich. In diesem Fall dient das Knochengewinde 5 dazu, dass das Verankerungselement 1 mit Hilfe eines Drehwerkzeugs wie eine herkömmliche Knochenschraube aus dem Knochen herausgeschraubt werden kann.

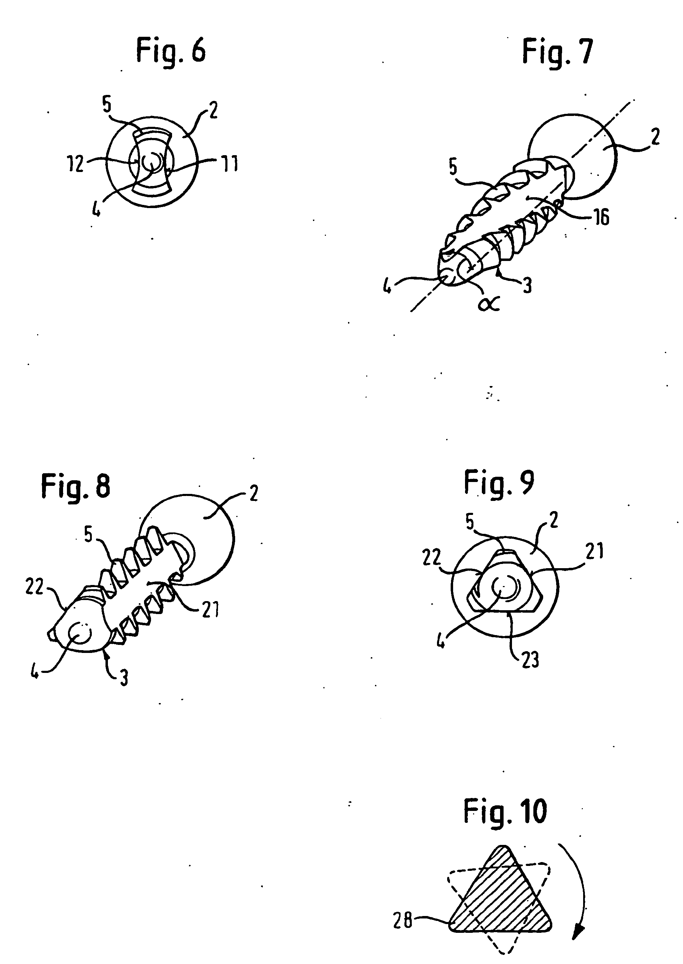

Fig. 6 zeigt eine zweite Ausführungsform der vorliegenden Erfindung. Anstelle der ebenen Flächen 6, 7 weist der Schaft 3 des Verankerungselements 1 zwei einander gegenüberliegende zur Schaftachse hin gewölbte Flächen 11, 12 auf, so dass sich ein Querschnitt des Schafts 3 mit konkaven Längsseiten ergibt. Ansonsten sind der Aufbau und der Einsatz des Verankerungselements 1 genauso wie bei der ersten Ausführungsform.

Durch die konkave Ausbildung der Flächen 11, 12 wird das Einwachsen von Knochenmaterial verbessert, wodurch sich ein festerer Halt des Verankerungselements 1 in dem Knochen ergibt.

Fig. 7 zeigt eine dritte Ausführungsform der vorliegenden Erfindung. Anstelle der Flächen 6, 7 bzw. 11, 12, die weist der Schaft 3 des Verankerungselements 1 zwei einander gegenüberliegende Flächen 16, 17 auf, die um einen vorbestimmten Winkel α um die Schaftachse tordiert sind, so dass sie einen Wendelabschnitt bilden. Ansonsten sind der Aufbau und der Einsatz des Verankerungselements 1 genauso wie bei der ersten Ausführungsform.

Auch wenn der vorbestimmten Winkel α in Fig. 7 zur Verdeutlichung annähernd als 90° dargestellt ist, liegt er vorzugsweise in einem Bereich von90°± 45°. Durch das leichte Tordieren der Flächen 16, 17 wird ebenfalls eine bessere Verankerung des Verankerungselements 1 in dem Knochen erzielt.

Auch die Flächen 16, 17 der dritten Ausführungsform können wie die Flächen 11, 12 der zweiten Ausführungsform konkav ausgebildet sein.

Mit Bezug auf Fig. 8 bis 10 wird ein Knochenverankerungselement nach einer vierten Ausführungsform der vorliegenden Erfindung beschrieben. Anstelle der zwei parallelen Flächen 6, 7 weist der Schaft 3 des Verankerungselements 1 drei gegeneinander um 120° versetzt ausgebildete Flächen 21, 22, 23 auf, so dass sich ein annähernd dreieckförmiger Querschnitt des Schafts 3 ergibt. Der Abstand der knochengewindefreien Flächen 21, 22, 23 von der Schaftachse ist kleiner als der Kernradius des Knochengewindes 5, so dass die Flächen 21, 22, 23 das Knochengewinde 5 unterbrechen. Ansonsten ist der Aufbau des Verankerungselements 1 genauso wie bei der ersten Ausführungsform.

Zum Einsetzen des Verankerungselements 1 nach der vierten Ausführungsform wird zunächst, wie am besten aus Fig. 10 ersichtlich, in den Knochen ein entsprechendes Dreieckloch 28 eingebracht. Das kann zum Beispiel durch Bohren eines Lochs und anschließende Formgebung mit einer Raspel geschehen. Alternativ dazu kann das Dreieckloch 28 auch mit Hilfe einer Querfräse oder Profilfräse erzeugt werden. Der Querschnitt des Dreiecklochs 28 entspricht annähernd dem Querschnitt des Schafts 3 oder ist etwas kleiner als dieser.

In das Dreieckloch 28 wird anschließend der Schaft 3 des Verankerungselements 1 durch Eindrücken oder Einklopfen so eingesetzt, dass die Dreieckseiten des Querschnitts durch den Schaft 3 annähernd mit den Dreieckseiten des Dreieckloch 28 übereinstimmt. In diesem Zustand sitzt das Verankerungselement 1 nur so fest in dem Dreieckloch 28, daß es durch Ziehen wieder entfernt werden kann.

Anschließend wird das Verankerungselement 1 mit einem Drehwerkzeug, das an oder in dem Kopf an- bzw. eingreift, um einen vorbestimmten Winkel gedreht, vorzugsweise um 60° (gestrichelte Linie in Fig. 10). Dadurch greifen die Zähne des Knochengewindes 5 in den Knochen ein und geben dem Verankerungselement 1 einen festen Sitz.

Durch Weiterdrehen bis zu einem Winkel von 120° oder Zurückdrehen in die Ausgangsstellung kann das Verankerungselement 1 bei Bedarf wieder gelöst und aus dem Knochen herausgezogen werden.

Auch in dieser Ausführungsform kann das Verankerungselement 1 nach längerer Zeit, wenn es bereits fest von Knochen umwachsen ist, mit Hilfe eines Drehwerkzeugs wie eine herkömmliche Knochenschraube aus dem Knochen herausgeschraubt werden.

Auch die Flächen 21, 22, 23 der vierten Ausführungsform können wie die Flächen 11, 12 der zweiten Ausführungsform konkav ausgebildet und/oder wie die Flächen 16, 17 der dritten Ausführungsform tordiert sein.

Das in der ersten bis vierten Ausführungsform beschriebene Knochenverankerungselement ist ideal geeignet zum Einsetzen in Pedikel eines Wirbelknochens, aber auch für alle anderen Knochen, in denen eine mechanische Vorrichtung verankert werden soll. Als Beispiel für eine Verbindung des Knochenverankerungselements mit einer externen Vorrichtung werden im folgenden zwei Arten der Verbindung eines Knochenverankerungselements mit einem Stab beschrieben.

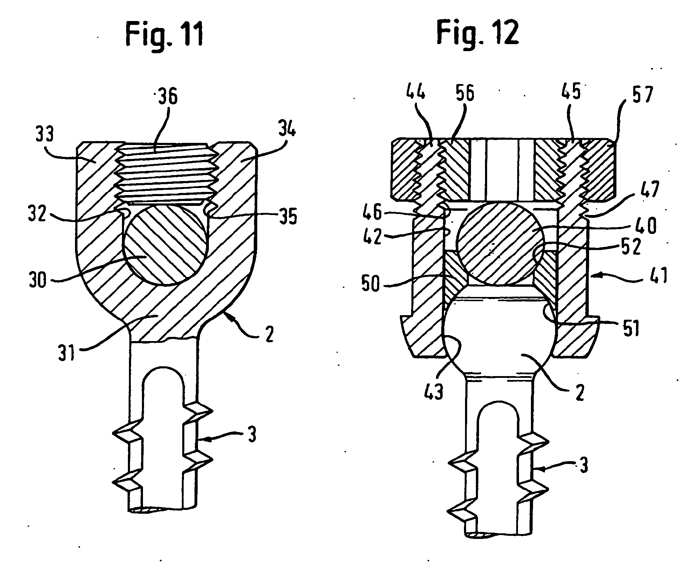

Fig. 11 ist eine schematische Darstellung einer monoaxialen Verbindung eines Knochenverankerungselements 1 mit einem Stab 30.

Der Kopf 2 des Knochenverankerungselements 1 ist als Aufnahmeteil 31 für den Stab 30 ausgebildet und weist eine U-förmige Ausnehmung 32 auf, durch die zwei freie Schenkel 33, 34 gebildet werden. Die Breite der eine U-förmigen Ausnehmung 32 entspricht in etwa dem Durchmesser des Stabs 30. Auf der Innenseite der Schenkel 33, 34 ist ein Innengewinde 35 vorgesehen.

Nach dem Einbringen des Knochenverankerungselements 1 in den Knochen wird der Stab 30 in die U-förmige Ausnehmung 32 eingeführt. Anschließend wird eine Schraube 36 soweit in das Innengewinde 35 eingeschraubt, dass der Stab 30 zwischen der Schraube 36 und dem Grund der U-förmigen Ausnehmung 32 fixiert wird.

Jede andere Art von monoaxialer Verbindung zwischen dem Schaft 3 und dem Aufnahmeteil ist möglich. Auch die Ausführung des Aufnahmeteils für den Stab ist nicht auf das dargestellte Ausführungsbeispiel beschränkt. Beipielsweise weist in einer Abwandlung das Aufnahmeteil kein Innengewinde auf. Das Aufnahmeteil wird dann mit einem Gewindestab verbunden und über seitlich angreifende Muttern fixiert.

Fig. 12 ist eine schematische Darstellung einer polyaxialen Verbindung eines Knochenverankerungselements 1 mit einem Stab 40.

Zur Verbindung ist ein im wesentlichen zylinderförmiges Aufnahmeteil 41 mit einer Längsbohrung 42 bereitgestellt. Der Durchmesser der Längsbohrung 42 ist geringfügig größer als der des kugelsegmentförmigen Kopfes 2 des Knochenverankerungselements 1. Die Längsbohrung 42 erstreckt sich von einem ersten Ende des Aufnahmeteils 41 aus in Richtung zu einem diesem gegenüberliegenden zweiten Ende. Zwischen dem zweiten Ende des Aufnahmeteils 41 und der Längsbohrung 42 ist eine kugelsegmentförmige Ausnehmung 43 ausgebildet, die annähernd den gleichen Durchmesser aufweist wie der kugelsegmentförmige Kopf 2 des Knochenverankerungselements 1.

Das Aufnahmeteil 41 weist weiter eine (nicht dargestellte) U-förmige Ausnehmung auf, deren Breite in etwa dem Durchmesser des Stabs 40 entspricht. Durch diese U-förmige Ausnehmung werden in dem Aufnahmeteil 41 zwei Schenkel 44, 45 gebildet. Auf der Innenseite der Schenkel 44, 45 ist ein Innengewinde 46 vorgesehen und auf der Außenseite ein Außengewinde 47.

Weiter ist ein hülsenförmiges Druckelement 50 vorgesehen. An einem Ende des hülsenförmiges Druckelements 50 ist eine kugelsegmentförmige Ausnehmung 51 ausgebildet, die annähernd den gleichen Durchmesser aufweist wie der kugelsegmentförmige Kopf 2 des Knochenverankerungselements 1, und an dem anderen Ende eine zylindersegmentförmige Ausnehmung 52, die annähernd den gleichen Durchmesser aufweist wie der Stab 40.

Zum Fixieren sind des Stabs und des Kopfs sind ferner eine in das Innengewinde 46 einschraubbare Innenschraube 56 und eine auf das Außengewinde 47 aufschraubbare Mutter 57 vorgesehen.

Beim Einsatz wird zunächst das Knochenverankerungselements 1 mit seinem Schaft 3 vom ersten Ende des Aufnahmeteils 41 her in dieses eingeführt, bis der Kopf 2 des Knochenverankerungselements 1 in dem kugelsegmentförmigen Abschnitt 43 gehalten wird. Anschließend wird das Knochenverankerungselement 1 in den Knochen eingebracht. Hierzu wird das Knochenverankerungselement 1 in ein im Knochen wie zuvor beschrieben gebildetes Loch eingedrückt oder geklopft.

Anschließend wird das hülsenförmige Druckelement 50 vom ersten Ende des Aufnahmeteils 41 her so in die Längsbohrung 42 eingeschoben, dass die kugelsegmentförmige Ausnehmung 51 auf den Kopf 2 des Knochenverankerungselements 1 zu liegen kommt. Dann wird der Stab 30 so in die U-förmige Ausnehmung eingeführt, dass er in die zylindersegmentförmige Ausnehmung 52 des Druckelements 50 zu liegen kommt.

Anschließend wird die Schraube 56 in das Innengewinde 46 eingeschraubt, so dass der Kopf 2 des Knochenverankerungselements 1 über das Druckelement 50 fixiert wird und gleichzeitig der Stab 40 von der Schraube 56 fixiert wird. Zum Schluss wird zum Sichern der Fixierung die Mutter 57 auf das Außengewinde 47 aufgeschraubt.

Die beschriebene polyaxiale Verbindung zwischen dem Kopf und dem Aufnahmeteil ist lediglich als Beispiel aufgeführt. Jede andere Art polyaxialer Verbindung ist denkbar.

Der in der ersten bis vierten Ausführungsform dargestellte kugelsegmentförmige Kopf ist besonders für die oben beschriebene polyaxiale Verbindung zwischen dem Knochenverankerungselement und der externen Vorrichtung geeignet. Das Knochenverankerungselement kann jedoch auch einen beliebigen anders geformten Kopf aufweisen, mit dem die externe zu verankernde Vorrichtung monoaxial oder polyaxial verbunden und/oder fixiert werden kann. Es kann z.B. auch einfach ein Senk- oder Linsenkopf vorgesehen sein, mit dem eine Platte an einem Knochen fixiert wird.

Der Schaft des Knochenverankerungselements kann alternativ auch ohne Spitze ausgebildet sein. Außerdem kann er zur Erleichterung des Einführens in den Knochen leicht konisch ausgebildet sein.

Statt über die gesamte Schaftlänge kann sich das Gewinde auch nur über einen vorbestimmten Abschnitt des Schafts erstrecken.

Die Anzahl der knochengewindefreien Flächen ist nicht auf 2 oder drei begrenzt. Alternativ dazu kann auch nur eine Fläche oder eine beliebige Anzahl von Flächen vorgesehen sein, die größer als drei ist.

Abstand der Flächen von der Schaftachse muss nicht kleiner sein als der Kernradius des Knochengewindes 5. Er kann auch gleich groß wie oder größer als der Kernradius gewählt werden. Auf jeden Fall muss er jedoch kleiner sein als der Abstand der Spitzen des Knochengewindes von der Schaftachse.

Die den konkaven Flächen 11, 12 entsprechenden Flächen müssen nicht über ihre gesamte Breite gewölbt sein. Es ist hinreichend, wenn nur ein Teil der Fläche gewölbt ist.

Zum Verbessern des Anwachsens des Knochens können die Flächen aufgeraut und / oder beschichtet sein, z.B. mit Hydroxylapatit.

Weiterhin kann das Knochenverankerungselement eine durch den Kopf führende und an der Spitze offene oder geschlossene Längsbohrung und von dieser seitwärts abzweigende und an den Seitenwänden des Schafts austretende Bohrungen aufweisen, durch die ein Medikament oder Knochenzement in das Befestigungsloch gespritzt werden kann.

In einer Weiterbildung ist das Knochenverankerungselement aus einer Formgedächtnislegierung wie z.B. Nitinol ausgebildet. Das Knochenverankerungselement kann dann z.B. so ausgebildet sein, dass es bei Raumtemperatur eine Fig. 1 entsprechende Form aufweist, bei der die knochengewindefreien Flächen eben sind und parallel zueinander liegen, und bei Körpertemperatur eine Fig. 7 entsprechende Form, bei der die Flächen verdreht zueinander sind.

Beim Einsetzen bei Raumtemperatur sorgen die parallelen Flächen dafür, dass das Knochenverankerungselement leicht einsetzbar ist. Im Körper verformt sich das Knochenverankerungselement dann so, dass die Flächen tordiert werden und ein verbesserter Halt in dem Knochen erzielt wird.

Das Knochenverankerungselement aus der Formgedächtnislegierung kann auch so ausgebildet sein, dass die Zähne des Knochengewindes bei Raumtemperatur kleiner sind als bei Körpertemperatur. Im Körper wachsen dann die Zähne des Knochengewindes, so dass ebenfalls ein verbesserter Halt in dem Knochen erzielt wird.

## Patentansprüche

1. Knochenverankerungselement (1) zum Verankern einer externen Vorrichtung in einem Knochen mit
einem mit der externen Vorrichtung verbindbaren Kopf (2) und
einem in einem Knochen zu verankernden Schaft (3), der in einem vorbestimmten Abschnitt ein Knochengewinde (5) aufweist,
**dadurch gekennzeichnet, dass**
der Schaft (3) wenigstens eine sich von einem ersten Ende bis zu einem zweiten Ende des vorbestimmten Abschnitts im wesentlichen in Richtung der Schaftachse erstreckende knochengewindefreie Fläche (6,7; 11,12; 16,17; 21,22,23) aufweist.

2. Knochenverankerungselement (1) nach Anspruch 1, **dadurch gekennzeichnet, dass** der Abstand der knochengewindefreien Fläche (6,7; 11,12; 16,17; 21,22,23) von der Schaftachse kleiner ist als der Abstand der Spitzen des Knochengewindes (5) von der Schaftachse.

3. Knochenverankerungselement (1) nach Anspruch 1 oder 2, **dadurch gekennzeichnet, dass** eine Mehrzahl von knochengewindefreien Flächen (6,7; 11,12; 16,17; 21,22,23) vorgesehen sind, vorzugsweise zwei oder drei.

4. Knochenverankerungselement (1) nach Anspruch 3, **dadurch gekennzeichnet, dass** die Mehrzahl von knochengewindefreien Flächen (6,7; 11,12; 16,17; 21,22,23) in Umfangsrichtung gleichmäßige Abstände aufweisen.

5. Knochenverankerungselement (1) nach einem der Ansprüche 1 bis 4, **dadurch gekennzeichnet, dass** wenigstens eine der knochengewindefreien Flächen als ebene Fläche (6,7; 21,22,23) ausgebildet ist.

6. Knochenverankerungselement (1) nach einem der Ansprüche 1 bis 5, **dadurch gekennzeichnet, dass** wenigstens eine der knochengewindefreien Flächen (11, 12) konkav ausgebildet ist.

7. Knochenverankerungselement (1) nach einem der Ansprüche 1 bis 6, **dadurch gekennzeichnet, dass** sich die knochengewindefreien Flächen (6,7; 21,22,23) parallel zu der Schaftachse erstrecken.

8. Knochenverankerungselement (1) nach einem der Ansprüche 1 bis 6, **dadurch gekennzeichnet, dass** sich die knochengewindefreien Flächen (16, 17) von dem ersten Ende zu dem zweiten Ende des vorbestimmten Abschnitts in Form eines Wendelabschnitts erstrecken.

9. Knochenverankerungselement (1) nach einem der Ansprüche 1 bis 8, **dadurch gekennzeichnet, dass** sich der vorbestimmte Abschnitt bis zu dem dem Kopf (2) gegenüberliegenden Ende des Schafts (3) erstreckt.

10. Knochenverankerungselement (1) nach einem der Ansprüche 1 bis 9, **dadurch gekennzeichnet, dass** an dem dem Kopf (2) gegenüberliegenden Ende des Schafts (3) eine Spitze (4) vorgesehen ist.

11. Knochenverankerungselement (1) nach einem der Ansprüche 1 bis 10, **dadurch gekennzeichnet, dass** wenigstens eine der knochengewindefreien Flächen (6,7; 11,12; 16,17; 21,22,23) zum Verbessern des Anwachsens des Knochens aufgeraut oder beschichtet ist.

12. Knochenverankerungselement (1) nach einem der Ansprüche 1 bis 11, **dadurch gekennzeichnet, dass** der Schaft (3) aus einer Formgedächtnislegierung ausgebildet ist.

13. Knochenverankerungselement (1) nach einem der Ansprüche 1 bis 12, **dadurch gekennzeichnet, dass** der Kopf (2) als Aufnahmeteil (31) zum Verbinden mit einem Stab (30) ausgebildet ist.

14. Knochenverankerungselement (1) nach einem-der Ansprüche 1 bis 13, **dadurch gekennzeichnet, dass** der Kopf (2) fest oder gelenkig mit dem Schaft (3) verbunden ist.

15. Knochenverankerungselement nach einem der Ansprüche 1 bis 14, **gekennzeichnet durch** eine **durch** den Kopf führende und an der Spitze offene oder geschlossene Längsbohrung.

16. Knochenverankerungselement nach Anspruch 15, **dadurch gekennzeichnet, dass** von der Längsbohrung seitwärts abzweigende und an den Seitenwänden des Schafts austretende Bohrungen vorgesehen sind.

## Claims

1. Bone anchoring element (1) for anchoring an external device in a bone comprising
a head (2) which is connectable to the external device and
a shaft (3) to be anchored in a bone which comprises a bone thread (5) in a predetermined section,
**characterized in that**
the shaft (3) comprises at least one bone thread-free surface (6,7; 11,12; 16,17; 21,22,23) that extends from a first end to a second end of the predetermined section essentially in the direction of the shaft axis.

2. Bone anchoring element (1) according to Claim 1, **characterized in that** the distance between the bone thread-free surface (6,7; 11,12; 16,17; 21,22,23) and the shaft axis is smaller than the distance between the tips of the bone thread (5) and the shaft axis.

3. Bone anchoring element (1) according to Claim 1 or 2, **characterized in that** a multitude of bone thread-free surfaces (6,7; 11,12; 16,17; 21,22,23) is provided, preferably two or three.

4. Bone anchoring element (1) according to Claim 3, **characterized in that** the multitude of bone thread-free surfaces (6,7; 11,12; 16,17; 21,22,23) are arranged at equal distances from each other in the circumferential direction.

5. Bone anchoring element (1) according to any of Claims 1 to 4, **characterized in that** at least one of the bone thread-free surfaces is provided as a level surface (6,7; 21,22,23).

6. Bone anchoring element (1) according to any of Claims 1 to 5, **characterized in that** at least one of the bone thread-free surfaces (11, 12) is provided to be concave in shape.

7. Bone anchoring element (1) according to any of Claims 1 to 6, **characterized in that** the bone thread-free surfaces (6,7; 21,22,23) extend parallel to the shaft axis.

8. Bone anchoring element (1) according to any of Claims 1 to 6, **characterized in that** the bone thread-free surfaces (16, 17) extend from the first end to the second end of the predetermined section in the form of a helical section.

9. Bone anchoring element (1) according to any of Claims 1 to 8, **characterized in that** the predetermined section extends to the end of the shaft (3) that is opposite to the head (2).

10. Bone anchoring element (1) according to any of Claims 1 to 9, **characterized in that** a tip (4) is provided at the end of the shaft (3) that is opposite to the head (2).

11. Bone anchoring element (1) according to any of Claims 1 to 10, **characterized in that** at least one of the bone thread-free surfaces (6,7; 11,12; 16,17; 21,22,23) is roughened or coated in order to improve the fusion to the bone.

12. Bone anchoring element (1) according to any of Claims 1 to 11, **characterized in that** the shaft (3) is made from a shape memory alloy.

13. Bone anchoring element (1) according to any of Claims 1 to 12, **characterized in that** the head (2) is provided as a receiving part (31) for connecting to a rod (30).

14. Bone anchoring element (1) according to any of Claims 1 to 13, **characterized in that** the head (2) is connected to the shaft (3) either fixedly or articulated.

15. Bone anchoring element according to one of Claims 1 to 14, **characterized by** a longitudinal bore which extends through the head and is open or closed at the tip.

16. Bone anchoring device according to Claim 15, **characterized in that** bores laterally branching off from the longitudinal bore and open on the side walls of the shaft are provided.

## Revendications

1. Elément d'ancrage osseux (1) pour l'ancrage d'un dispositif externe dans un os avec une tête (2) pouvant être reliée au dispositif externe et une tige (3) à ancrer dans un os, qui présente un filetage osseux (5) dans une partie prédéfinie, **caractérisé en ce que** la tige (3) présente au moins une surface (6, 7 ; 11, 12 ; 16, 17 ; 21, 22, 23) sans filetage osseux s'étendant depuis une première extrémité jusqu'à une seconde extrémité de la partie prédéfinie essentiellement en direction de l'axe de la tige.

2. Elément d'ancrage osseux (1) selon la revendication 1, **caractérisé en ce que** la distance de la surface (6, 7 ; 11, 12 ; 16, 17 ; 21, 22, 23) sans filetage osseux à l'axe de la tige est inférieure à la distance des extrémités du filetage osseux (5) à l'axe de la tige.

3. Elément d'ancrage osseux (1) selon la revendication 1 ou 2, **caractérisé en ce qu'**il est prévu une pluralité, de préférence deux ou trois, de surfaces (6, 7 ; 11, 12 ; 16, 17 ; 21, 22, 23) sans filetage osseux.

4. Elément d'ancrage osseux (1) selon la revendication 3, **caractérisé en ce que** la pluralité de surfaces (6, 7 ; 11, 12 ; 16, 17 ; 21, 22, 23) sans filetage d'os présente des espacements uniformes dans le sens périphérique.

5. Elément d'ancrage osseux (1) selon l'une quelconque des revendications 1 à 4, **caractérisé en ce qu'**au moins l'une des surfaces sans filetage osseux est conçue comme une surface (6, 7 ; 21, 22, 23) plane.

6. Elément d'ancrage osseux (1) selon l'une quelconque des revendications 1 à 5, **caractérisé en ce qu'**au moins l'une des surfaces (11, 12) sans filetage osseux est conçue concave.

7. Elément d'ancrage osseux (1) selon l'une quelconque des revendications 1 à 6, **caractérisé en ce que** les surfaces (6, 7 ; 21, 22, 23) sans filetage osseux s'étendent parallèlement à l'axe de la tige.

8. Elément d'ancrage osseux (1) selon l'une quelconque des revendications 1 à 6, **caractérisé en ce que** les surfaces (16, 17) sans filetage osseux s'étendent de la première extrémité jusqu'à la seconde extrémité de la partie prédéfinie sous la forme d'une partie hélicoïdale.

9. Elément d'ancrage osseux (1) selon l'une quelconque des revendications 1 à 8, **caractérisé en ce que** la partie prédéfinie s'étend jusqu'à l'extrémité, opposée à la tête (2), de la tige (3).

10. Elément d'ancrage osseux (1) selon l'une quelconque des revendications 1 à 9, **caractérisé en ce que** une pointe (4) est prévue sur l'extrémité, opposée à la tête (2), de la tige (3).

11. Elément d'ancrage osseux (1) selon l'une quelconque des revendications 1 à 10, **caractérisé en ce qu'**au moins une des surfaces (6, 7 ; 11, 12 ; 16, 17 ; 21, 22, 23) sans filetage osseux est rendue rugueuse ou revêtue pour améliorer la croissance de l'os.

12. Elément d'ancrage osseux (1) selon l'une quelconque des revendications 1 à 11, **caractérisé en ce que** la tige (3) est conçue à base d'un alliage à mémoire de forme.

13. Elément d'ancrage osseux (1) selon l'une quelconque des revendications 1 à 12, **caractérisé en ce que** la tête (2) est conçue comme partie de réception (31) pour la liaison avec une barre (30).

14. Elément d'ancrage osseux (1) selon l'une quelconque des revendications 1 à 13, **caractérisé en ce que** la tête (2) est reliée de façon fixe ou articulée avec la tige (3).

15. Elément d'ancrage osseux selon l'une quelconque des revendications 1 à 14, **caractérisé par** un alésage longitudinal passant à travers la tête et ouvert ou fermé sur l'extrémité.

16. Elément d'ancrage osseux selon la revendication 15, **caractérisé en ce que** des alésages déviant de l'alésage longitudinal sur le côté et sortant sur les parois latérales de la tige sont prévus.
